# EUROPEAN PATENT SPECIFICATION

(11) **EP 4 160 276 B1**
(45) Date of publication and mention of the grant of the patent: **13.08.2025**
(21) Application number: 21813817.0
(22) Date of filing: 24.05.2021
(51) Int. Cl.: G01N 23/046, A61B 6/03

(54) **CT SCANNING DEVICE**
CT-SCANNING-EINRICHTUNG
DISPOSITIF DE TOMODENSITOMÉTRIE

(30) Priority: 29.05.2020 CN 202010482145
(43) Date of publication of application: 05.04.2023
(73) Proprietor: Tsinghua University, Haidian District, Beijing 100084 (CN); Nuctech Company Limited, TongFang Building Shuangqinglu Haidian District Beijing 100084 (CN)
(72) Inventor: CHEN, Zhiqiang, Beijing 100084 (CN); ZHANG, Li, Beijing 100084 (CN); HUANG, Qingping, Beijing 100084 (CN); HONG, Mingzhi, Beijing 100084 (CN); ZHANG, Liguo, Beijing 100084 (CN); LI, Guipei, Beijing 100084 (CN)
(74) Representative: Gunzelmann, Rainer
(86) International application number: PCT/CN2021/095512
(87) International publication number: WO 2021/238861

(56) References cited:
- WO-A1-2019/077544
- CN-A- 104 490 414
- CN-A- 111 856 600
- CN-U- 203 510 301
- CN-U- 204 306 836
- CN-U- 205 328 313
- CN-U- 206 499 473
- CN-U- 206 499 473
- CN-Y- 2 475 117
- US-A- 5 982 844
- US-A1- 2018 177 473
- US-B1- 7 020 233

## Description

### TECHNICAL FIELD

The embodiments of the present disclosure relate to a CT scanning apparatus, and in particular to a CT scanning apparatus driving a slip ring through a transmission belt.

### BACKGROUND

A traditional CT scanning apparatus mainly includes: a scanning device, a computer system, a power supply and an auxiliary apparatus. The scanning device includes a slip ring (rotating gantry), an X-ray tube and a detector which are mounted on the slip ring. An X-ray beam generated by the X-ray tube performs a transverse scanning to an inspected object such as a human body or a cargo through a collimator. Each detector receives an attenuation signal from an X-ray penetrating a human tissue or a cargo, converts the attenuation signal to an electrical signal, and then the electrical signal is converted into a digital signal (i.e., the original data) by an analog-to-digital conversion, and the digital signal is then stored in a memory. A magnitude of attenuation of the X-ray penetrating the inspected object is a function of a density of a substance through which a radiation beam passes. An attenuated X-ray is detected, and an X-ray photo image of the inspected object is generated to illustrate a result of the inspection.

In an existing CT scanning apparatus, a single large bearing is used as a rotating support for a slip ring. The slip ring, the X-ray tube and the detector are rotatably mounted on the large bearing, and a driving mechanism drives the slip ring to rotate through a multi-ribbed belt. A transmission channel for transmitting an inspected object passes through the large bearing and the slip ring, and the inspected object is detected while moving in the transmission channel. Generally, the slip ring is located inside a closed endless belt enclosed by the multi-ribbed belt. Since the multi-ribbed belt is a consumable part, it is required to be replaced within a certain period of time. During removal or mounting of the multi-ribbed belt, the ribbed belt is required to be removed from the slip ring or sleeved onto the slip ring from a side of the transmission channel. In this case, the operation of replacing the multi-ribbed belt is cumbersome and a

### SUMMARY

The objective of the present disclosure is to solve at least one aspect of the above problems and defects in the related art.

According to an embodiment in an aspect of the present disclosure, a CT scanning apparatus is provided, including: a support frame; a slip ring rotatably mounted on the support frame; a driving mechanism mounted on the support frame, wherein the driving mechanism comprises a driving wheel; a guide wheel mounted on the support frame; and an endless belt enclosing a closed periphery, wherein the endless belt is in contact with the slip ring, the driving wheel and the guide wheel, so that the driving wheel drives the slip ring and the guide wheel to rotate through the endless belt, wherein the slip ring is in contact with the endless belt on an outer side of the periphery.

According to an embodiment of the present disclosure, the driving wheel is in contact with the endless belt on the outer side of the periphery.

According to an embodiment of the present disclosure, the guide wheel comprises a plurality of guide wheels arranged around the slip ring, and the plurality of guide wheels are in contact with the endless belt on an inner side of the periphery.

According to an embodiment of the present disclosure, one of the plurality of guide wheels serves as a tensioning wheel configured to tension the endless belt.

According to an embodiment of the present disclosure, the CT scanning apparatus further includes a tensioning wheel configured to tension the endless belt.

According to an embodiment of the present disclosure, the support frame comprises: an outer frame; and an inner frame having a cylinder shape, wherein the inner frame is fixed in the outer frame through a plurality of connecting members, the slip ring is rotatably mounted in the inner frame through a bearing mechanism and protruding axially from the inner frame to form a protruding portion, and the protruding portion is combined with the endless belt.

According to an embodiment of the present disclosure, the driving mechanism further comprises: a supporting seat mounted on the outer frame and facing the inner frame; and a motor mounted on an outer side of the supporting seat opposite to the inner frame, wherein an output shaft of the motor passes through the supporting seat and extends parallel to an axis of the slip ring, and the driving wheel is mounted on the output shaft on an inner side of the supporting seat.

According to an embodiment of the present disclosure, the plurality of guide wheels comprise a first guide wheel, a second guide wheel, a third guide wheel and a fourth guide wheel respectively located at vertexes of a quadrangle, the endless belt between the first guide wheel and the second guide wheel is combined with the driving wheel, and the first guide wheel and the second guide wheel are rotatably mounted on the inner side of the supporting seat.

According to an embodiment of the present disclosure, the endless belt between the first guide wheel and the second guide wheel is combined with the slip ring, and the first guide wheel is rotatably mounted on one of the connecting members.

According to an embodiment of the present disclosure, the fourth guide wheel is rotatably mounted on another one of the connecting members.

According to an embodiment of the present disclosure, the endless belt is made of a multi-ribbed belt, and the protruding portion of the slip ring is provided with a sheave cooperating with the multi-ribbed belt.

According to an embodiment of the present disclosure, an X-ray tube configured to emit an X-ray and a detector array radially opposite to the X-ray tube are mounted on the slip ring.

According to an embodiment of the present disclosure, the endless belt is in contact with a portion of an outer circumference of the slip ring located between the X-ray tube and the detector array.

### BRIEF DESCRIPTION OF THE DRAWINGS

FIG 1 shows a simplified schematic diagram of a CT scanning apparatus according to an exemplary embodiment of the present disclosure;
FIG 2 shows a principle schematic diagram of a CT scanning apparatus according to an exemplary embodiment of the present disclosure;
FIG 3 shows a simplified stereo diagram of a CT scanning apparatus according to an exemplary embodiment of the present disclosure;
FIG 4 shows a front view of the CT scanning apparatus shown in FIG 3, wherein a supporting seat and a motor of a driving mechanism are not shown;
FIG 5 shows a side view of the CT scanning apparatus shown in FIG. 4, including a cross-sectional view taken along line A-A of FIG 4;
FIG 6 shows an enlarged schematic diagram of part A shown in FIG. 5;
FIG 7 shows an enlarged schematic diagram of part B shown in FIG 5;
FIG 8 shows a simplified schematic diagram of a driving manner of a slip ring according to an exemplary embodiment of the present disclosure;
FIG 9 shows a simplified schematic diagram of a driving manner of a slip ring according to another exemplary embodiment of the present disclosure; and
FIG 10 shows a simplified schematic diagram of a driving manner of a slip ring according to yet another exemplary embodiment of the present disclosure.

### DETAILED DESCRIPTION OF EMBODIMENTS

Technical solutions in the embodiments of the present disclosure will be clearly and completely described below with reference to the accompanying drawings in the embodiments of the present disclosure. Obviously, the described embodiments are only a part, not all of the embodiments of the present disclosure. The following description of at least one exemplary embodiment is actually illustrative only and is in no way intended to limit the present disclosure and an application or use of the present disclosure. Based on the embodiments of the present disclosure, all other embodiments obtained by those of ordinary skill in the art without creative work fall within the protection scope of the present disclosure.

In the following detailed descriptions, for ease of explanation, various specific details are set forth in order to provide a comprehensive understanding of the embodiments of the present disclosure. Obviously, however, one or more embodiments may be implemented without these specific details. In other circumstances, well-known structures and devices are shown in a diagram form to simplify the drawings. Technologies, methods, and apparatuses known to those of ordinary skill in the related art may not be discussed in detail, but where appropriate, such technologies, methods, and apparatuses should be considered as a part of the authorized specification.

In the descriptions of the present disclosure, it should be understood that orientations or positional relationships indicated by orientation words such as "front, rear, upper, lower, left, right", "transverse, vertical, perpendicular, horizontal" and "top, bottom" are usually orientations or positional relationships based on the drawings, and are based on a travel direction of a vehicle. They are only used for ease of describing the present disclosure and simplifying the description. Unless stated in contrary, these orientation words do not indicate and imply that a device or an element referred to necessarily has a specific orientation or is configured and operated in a specific orientation. Therefore, they should not be construed as a limitation on the protection scope of the present disclosure. The orientation words "inner" and "outer" refer to an inside and an outside relative to a profile of each component itself.

In the descriptions of the present disclosure, it should be understood that words such as "first" and "second" used to define components are only for ease of distinguishing the corresponding components. Unless otherwise stated, the above-mentioned words have no special meaning, and therefore, they may not be construed as a limitation on the protection scope of the present disclosure.

According to a general inventive concept of the present disclosure, there is provided a CT scanning apparatus, including: a support frame; a slip ring rotatably mounted on the support frame; a driving mechanism mounted on the support frame and including a driving wheel; a plurality of guide wheels mounted on the support frame; and an endless belt enclosing a closed periphery, wherein the endless belt is in contact with the slip ring, the driving wheel and the guide wheel, so that the driving wheel drives the slip ring and the guide wheel to rotate through the endless belt. The slip ring is in contact with the endless belt on an outer side of the periphery.

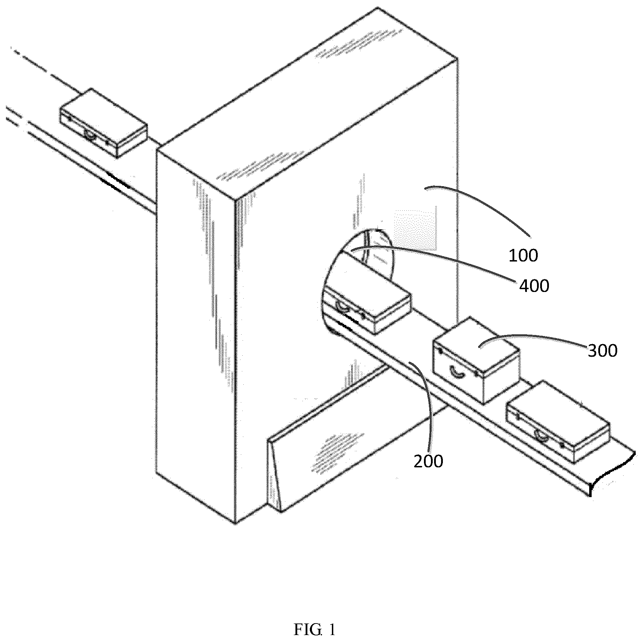

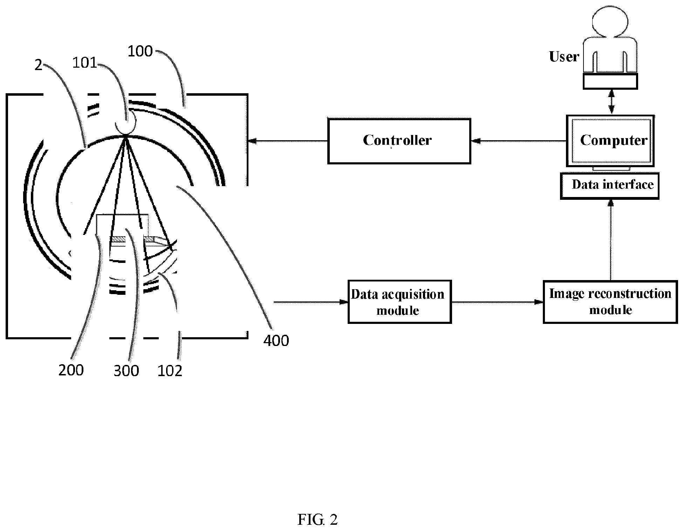

FIG 1 shows a simplified schematic diagram of a CT scanning apparatus according to an exemplary embodiment of the present disclosure. FIG 2 shows a principle schematic diagram of a CT (computed tomography) scanning apparatus according to an exemplary embodiment of the present disclosure.

In an exemplary embodiment, referring to FIG 1 and FIG 2, a CT scanning apparatus 100 is suitable for inspecting a presence of prohibited items such as drugs, explosives, and flammables in an object 300 such as a package, a suitcase and a handbag at a place such as a station, an airport, a dock and the like. The CT scanning apparatus 100 includes: an inspection channel 400; a transmission device 200 transmitting an inspected object 300 in the inspection channel 400; and a scanning device configured to inspect the object 300 transmitted by the transmission device 200. The transmission device 200 includes a transmission belt 201 for carrying the inspected object and a driving roller 202 for driving the transmission belt to move.

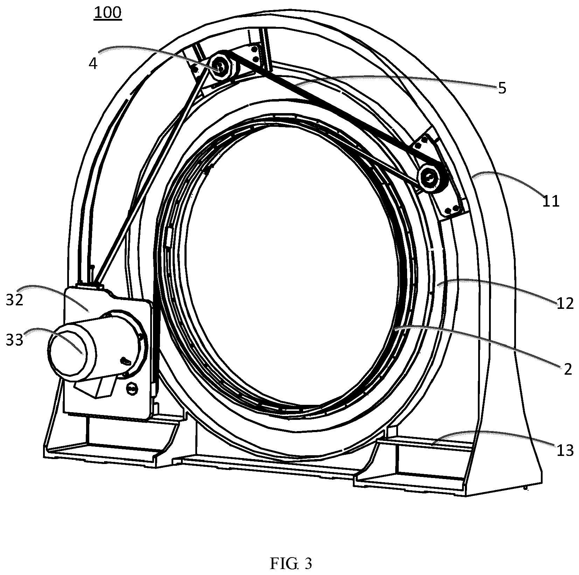

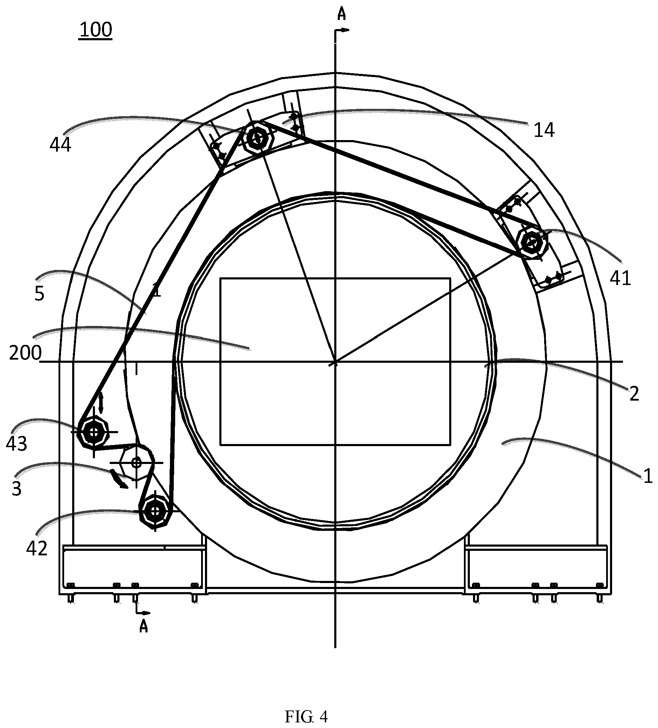

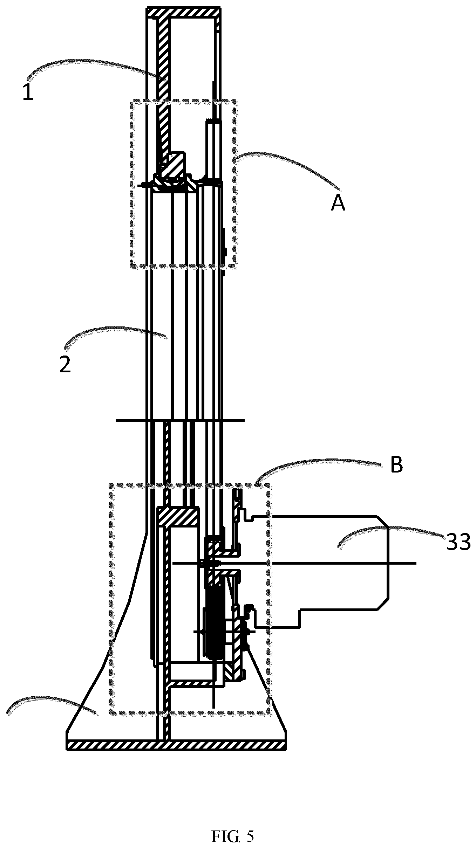

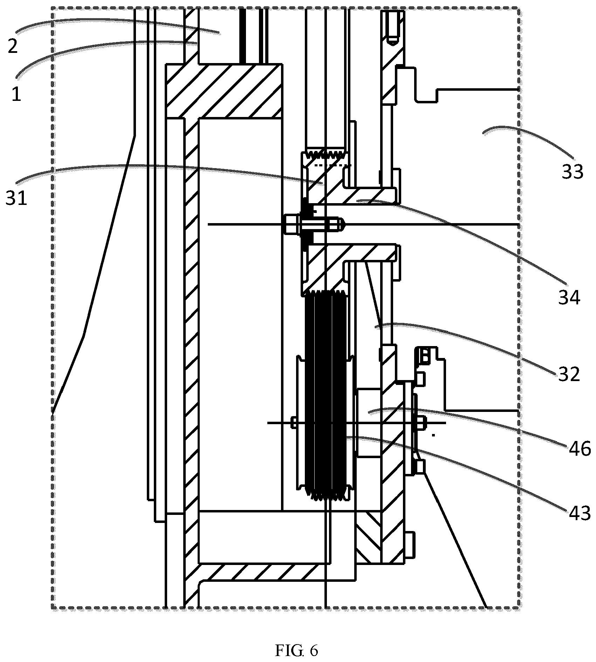

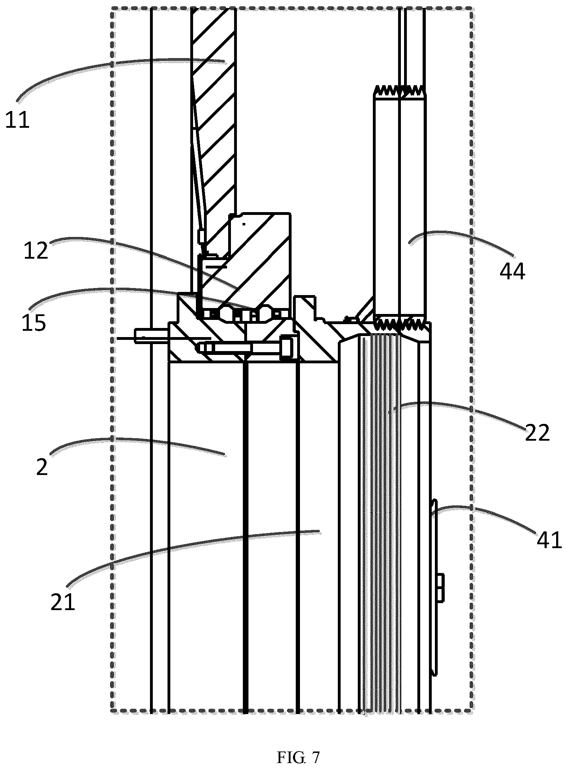

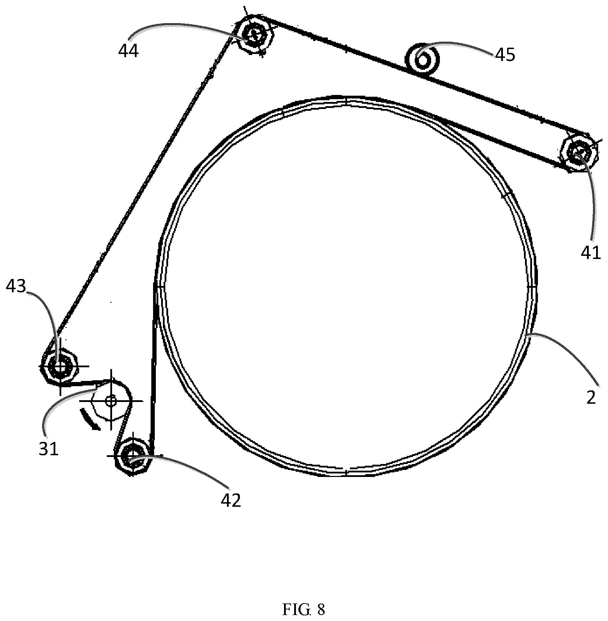

FIG 3 shows a simplified stereo diagram of a CT scanning apparatus of an exemplary embodiment of the present disclosure. FIG 4 shows a front view of the CT scanning apparatus shown in FIG 3, wherein a supporting seat and a motor of a driving mechanism are not shown. FIG 5 shows a side view of the CT scanning apparatus shown in FIG 4, including a cross-sectional view taken along line A-A of FIG 4. FIG 6 shows an enlarged schematic diagram of part A shown in FIG. 5. FIG 7 shows an enlarged schematic diagram of part B shown in FIG 5. FIG 8 shows a simplified schematic diagram of a driving manner of a slip ring according to an exemplary embodiment of the present disclosure.

In an exemplary embodiment, referring to FIG 1 to FIG 8, the CT scanning apparatus 100 further includes: a support frame 1 having an outer profile of a substantially arched shape and being supported on a pedestal 13; a slip ring 2 rotatably supported on the support frame 1, an inspection passage 400 penetrating the slip ring 2; a driving mechanism 3 mounted on the support frame 1 and including a driving wheel 31; a plurality of guide wheels 4 mounted on the support frame 1; and an endless belt 5 enclosing a closed periphery, wherein the endless belt 5 is in contact with the slip ring 2, the driving wheel 31 and the guide wheels 4, so that the driving wheel drives 31 the slip ring 2 and the guide wheels 4 to rotate through the endless belt 5. The slip ring 2 is in contact with the endless belt 5 on an outer side of the periphery. That is, the slip ring 2 is located on an inner side of a closed annular space enclosed by the endless belt 5. The scanning device includes the slip ring (rotating gantry) 2, and an X-ray tube 101 and a detector array 102 which are mounted on the slip ring 2.

During an inspection of the object 300, a controller receives an operation instruction input by a user through a computer at a workstation, and controls an action of the driving mechanism 3 according to the operation instruction. The slip ring drives the X-ray tube and the detector 102 to rotate under a drive of the driving mechanism 3, while the X-ray tube 101 may generate an X-ray beam under a control of the controller. The X-ray beam penetrates the inspected object 300 moving on the transmission device 200 and is irradiated onto a detector array 102. The detector array 102 converts the X-ray beam received into an electrical signal and transmits the electrical signal to a data acquisition module. An image recognition module receives data of the data acquisition module, reconstructs the data received and generates image data. The image data generated is transmitted to the computer for recognition and inspection of the inspected object.

In an exemplary embodiment, as shown in FIG 4 and FIG 8, the driving wheel 31 is in contact with the endless belt 5 on the outer side of the periphery. In this way, the endless belt is in contact with at least a portion of an outer circular ring of the driving wheel without being sleeved on the driving wheel, which facilitates an operation of combing the endless belt with the slip ring or removing the endless belt from the slip ring.

In an exemplary embodiment, as shown in FIG 4 and FIG 8, the guide wheels include a plurality of guide wheels arranged around the slip ring. The plurality of guide wheels are in contact with the endless belt 5 on an inner side of the periphery, and are respectively located at vertexes of a polygon. It may be understood that each guide wheel is rotatably connected to a fixed shaft through a bearing mechanism.

Further, one of the plurality of guide wheels serves as a tensioning wheel configured to tension the endless belt 5. In another exemplary embodiment, the CT scanning apparatus 100 further includes a tensioning wheel 45 configured to tension the endless belt 5. By providing the tensioning wheel, the endless belt 5 may be kept in close contact with the driving wheel 31 and the slip ring 2, and a friction force between the endless belt and the driving wheel as well as the slip ring is increased.

In an exemplary embodiment, as shown in FIG 3 to FIG 7, the support frame 1 includes: an outer frame 11 mounted on the pedestal 13; and an inner frame 12 having a cylinder shape, wherein the inner frame 12 is fixed in the outer frame 11 through a plurality of connecting members 14. The slip ring 2 is rotatably mounted in the inner frame 12 through a bearing mechanism 15 and protrudes axially from the inner frame 12 so as to form a protruding portion 21, and the protruding portion 21 is combined with the endless belt 5.

In an exemplary embodiment, as shown in FIG 3 to FIG. 6, the driving mechanism 3 further includes: a supporting seat 32 mounted on the outer frame 11 and facing the inner frame 12; and a motor 33 mounted on an outer side of the supporting seat 32 opposite to the inner frame 12, wherein an output shaft 34 of the motor passes through the supporting seat 32 and extends parallel to an axis of the slip ring 2, and the driving wheel 31 is mounted on the output shaft 34 on an inner side of the supporting seat 32.

In an exemplary embodiment, as shown in FIG 3, FIG 4 and FIG 8, the plurality of guide wheels include a first guide wheel 41, a second guide wheel 42, a third guide wheel 43 and a fourth guide wheel 44 respectively located at vertexes of a quadrangle. An endless belt 5 between the second guide wheel 42 and the third guide wheel 43 is combined with the driving wheel 31, and the second guide wheel 42 and the third guide wheel 43 are rotatably mounted on the inner side of the supporting seat 32. In this way, there is a predetermined space between the inner frame 12 and the supporting seat 32, and the predetermined space may be used to accommodate the driving wheel 31, the second guide wheel 42 and the third guide wheel 43, so that a thickness of the CT scanning apparatus 100 may be reduced.

In an exemplary embodiment, as shown in FIG 3, FIG 4, FIG 7 and FIG 8, the endless belt 5 between the first guide wheel 41 and the second guide wheel 42 is combined with the slip ring 2. That is, the second guide wheel 42 is configured to abut the endless belt 5 against the driving wheel 31 and the slip ring 2. The first guide wheel 41 is rotatably mounted on one of the connecting members 14. The fourth guide wheel 44 is rotatably mounted on another one of the connecting members. That is, the first guide wheel 41 and the fourth guide wheel 44 are respectively rotatably mounted on the two connecting members 14 between the outer frame 11 and the inner frame 12, and rotation shafts of the first guide wheel 41 and the fourth guide wheel 44 are parallel to the axis of the slip ring 2, and do not exceed the protruding portion 21 of the slip ring 2, so that the thickness of the CT scanning apparatus 100 is reduced.

According to an exemplary embodiment, as shown in FIG. 6 to FIG 8, the endless belt 5 is made of a multi-ribbed belt, and the protruding portion 21 of the slip ring 2 is provided with a sheave 22 cooperating with the multi-ribbed belt. A plurality of protruding tooth-shaped portions of the multi-ribbed belt cooperate with a plurality of annular grooves of the sheave 22. This may increase a friction between the multi-ribbed belt and the sheave, and prevent the multi-ribbed belt from sliding with respect to the slip ring. The slip ring 2 is in contact with a side of the multi-ribbed belt provided with the tooth shaped portions, and the guide wheels are in contact with a side of the multi-ribbed belt opposite to the tooth-shaped portions. That is, the side of the multi-ribbed belt provided with the tooth shaped portions is arranged toward the outside. It may be understood that similar sheaves are also provided on the guide wheel 4 and the driving wheel 31. In an alternative embodiment, a cross section of the endless belt 5 is substantially triangular or trapezoidal. In another alternative embodiment, the endless belt 5 is a flat belt.

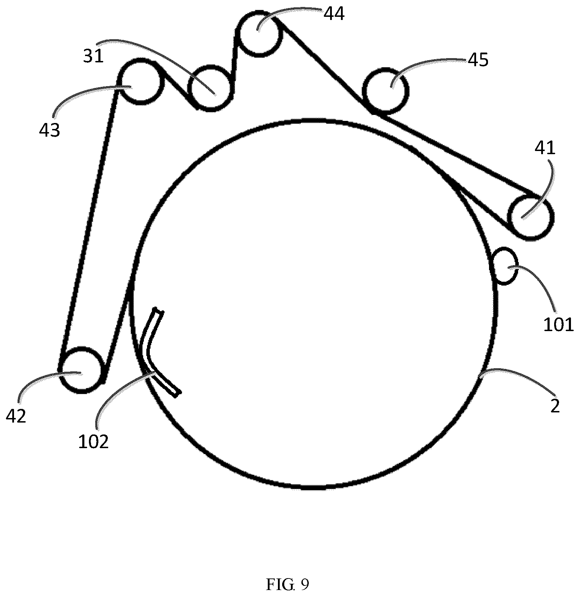

FIG 9 shows a simplified schematic diagram of a driving manner of a slip ring according to another exemplary embodiment of the present disclosure.

In an exemplary embodiment, as shown in FIG. 9, the guide wheels include a first guide wheel 41, a second guide wheel 42, a third guide wheel 43 and a fourth guide wheel 44 which are arranged around the slip ring. The slip ring is in contact with the endless belt on the outer side of the closed periphery enclosed by the endless belt 5. The first guide wheel 41, the second guide wheel 42, the third guide wheel 43 and the fourth guide wheel 44 are in contact with the endless belt 5 on the inner side of the periphery and are respectively located at vertexes of a quadrilateral. The CT scanning apparatus 100 further includes a tensioning wheel 45 configured to tension the endless belt 5. The third guide wheel 43 and the fourth guide wheel 44 are rotatably mounted on the support frame, and the endless belt 5 between the third guide wheel 43 and the fourth guide wheel 44 is combined with the driving wheel 31. The endless belt 5 between the first guide wheel 41 and the second guide wheel 42 is combined with the slip ring 2.

In an exemplary embodiment, as shown in FIG 2 and FIG 9, an X-ray tube 101 configured to emit an X-ray and a detector array 102 radially opposite to the X-ray tube 101 are mounted on the slip ring 2. The X-ray tube 101 and the detector array 102 are driven by the driving mechanism 3 to rotate around the transmission channel 100 so as to perform an X-ray scanning inspection on the inspected object 300 moving in the transmission channel. The endless belt 5 is in contact with a portion of an outer circumference of the slip ring 2 between the X-ray tube 101 and the detector array 102. That is, the endless belt 5 is not in contact with a portion of the outer circumference of the slip ring 2 where the X-ray tube 101 and the detector array 102 are arranged. In this way, the removal and mounting of the endless belt will not affect the X-ray tube 101.

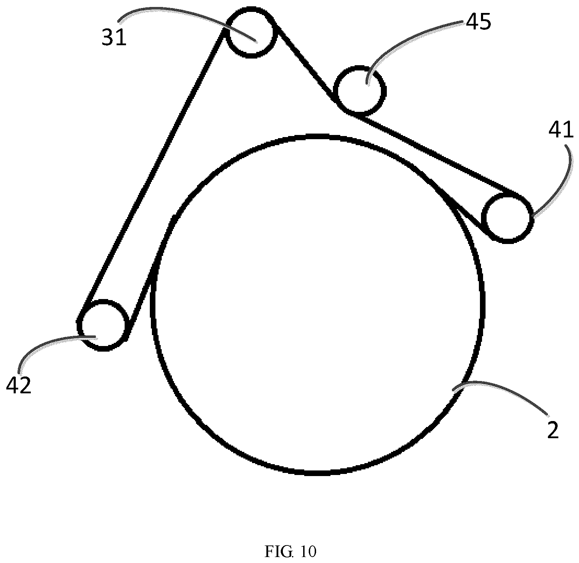

FIG 10 shows a simplified schematic diagram of a driving manner of a slip ring according to yet another exemplary embodiment of the present disclosure.

In an exemplary embodiment, as shown in FIG 10, the guide wheels include a first guide wheel 41 and a second guide wheel 42 which are arranged around the slip ring, and the slip ring is in contact with the endless belt on the outer side of the closed periphery enclosed by the endless belt 5. The first guide wheel 41, the second guide wheel 42 and the driving wheel 31 are in contact with the endless belt 5 on the inner side of the periphery, and are respectively located at vertexes of a triangle. The CT scanning apparatus 100 further includes a tensioning wheel 45 configured to tension the endless belt 5. Those skilled in the art may understand that different numbers of guide wheels may be arranged and the guide wheels may be arranged in different positions as required, as long as the slip ring is in contact with the endless belt 5 on the outer side of the closed periphery enclosed by the endless belt 5.

According to the CT scanning apparatus of the embodiments of the present disclosure, the slip ring 2 is in contact with the endless belt 5 on the outer side of the periphery, and the endless belt 5 is in contact with the slip ring 2, the driving wheel 31 and the guide wheels 4, so that the driving wheel 31 drives the slip ring 2 and the guide wheels 4 to rotate through the endless belt 5. An outer diameter of the driving wheel 31 as a drive wheel may be very small, while an outer diameter of the slip ring as a driven wheel remains unchanged. By arranging a plurality of guide wheels 4, the slip ring may be driven to rotate around the axis. The slip ring is in contact with the endless belt on the outer side of the periphery, and the endless belt is in contact with at least a portion of a circular ring outside the slip ring without being sleeved on the slip ring. In this way, during mounting, repairing or replacing the endless belt, other components, such as a protective housing, at an outer periphery of the slip ring are not required to be completely removed, so that an operation time of combining the endless belt with the slip ring or removing the endless belt from the slip ring may be significantly shortened. At the same time, due to an increase of the number of the guide wheels, the endless belt becomes longer, which is beneficial to reduce a wear amount of the endless belt and prolong a life of the endless belt.

Those skilled in the art may understand that the embodiments described above are all exemplary, and those skilled in the art may make improvements thereto. Structures described in various embodiments may be freely combined without a structure or principle conflict.

## Claims

1. A CT scanning apparatus (100), comprising:
a support frame (1);
a slip ring (2) rotatably mounted on the support frame;
a driving mechanism (3) mounted on the support frame, wherein the driving mechanism comprises a driving wheel (31);
a guide wheel (4) mounted on the support frame; and
an endless belt (5) enclosing a closed periphery, wherein the endless belt is in contact with the slip ring, the driving wheel and the guide wheel, so that the driving wheel drives the slip ring and the guide wheel to rotate through the endless belt,
wherein the slip ring is in contact with the endless belt on an outer side of the periphery.

2. The CT scanning apparatus according to claim 1, wherein the driving wheel is in contact with the endless belt on the outer side of the periphery.

3. The CT scanning apparatus according to claim 2, wherein the guide wheel comprises a plurality of guide wheels arranged around the slip ring, and the plurality of guide wheels are in contact with the endless belt on an inner side of the periphery.

4. The CT scanning apparatus according to claim 3, wherein one of the plurality of guide wheels serves as a tensioning wheel configured to tension the endless belt.

5. The CT scanning apparatus according to claim 3, further comprising a tensioning wheel (45) configured to tension the endless belt.

6. The CT scanning apparatus according to any one of claims 3-5, wherein the support frame comprises:
an outer frame (11); and
an inner frame (12) having a cylinder shape, wherein the inner frame is fixed in the outer frame through a plurality of connecting members, the slip ring is rotatably mounted in the inner frame through a bearing mechanism and protruding axially from the inner frame to form a protruding portion (21), and the protruding portion is combined with the endless belt.

7. The CT scanning apparatus according to claim 6, wherein the driving mechanism further comprises:
a supporting seat (32) mounted on the outer frame and facing the inner frame; and
a motor (33) mounted on an outer side of the supporting seat opposite to the inner frame, wherein an output shaft (34) of the motor passes through the supporting seat and extends parallel to an axis of the slip ring, and the driving wheel is mounted on the output shaft on an inner side of the supporting seat.

8. The CT scanning apparatus according to claim 7, wherein
the plurality of guide wheels comprise a first guide wheel (41), a second guide wheel (42), a third guide wheel (43) and a fourth guide wheel (44) respectively located at vertexes of a quadrangle,
the endless belt between the first guide wheel and the second guide wheel is combined with the driving wheel, and
the first guide wheel and the second guide wheel are rotatably mounted on the inner side of the supporting seat.

9. The CT scanning apparatus according to claim 8, wherein the endless belt between the first guide wheel and the second guide wheel is combined with the slip ring, and
the first guide wheel is rotatably mounted on one of the connecting members.

10. The CT scanning apparatus according to claim 9, wherein the fourth guide wheel is rotatably mounted on another one of the connecting members.

11. The CT scanning apparatus according to any one of claims 6-10, wherein the endless belt is made of a multi-ribbed belt, and the protruding portion of the slip ring is provided with a sheave (22) cooperating with the multi-ribbed belt.

12. The CT scanning apparatus according to any one of claims 1-11, wherein an X-ray tube configured to emit an X-ray and a detector array radially opposite to the X-ray tube are mounted on the slip ring.

13. The CT scanning apparatus according to claim 12, wherein,
the endless belt is in contact with a portion of an outer circumference of the slip ring located between the X-ray tube and the detector array.

## Patentansprüche

1. Eine CT-Scanvorrichtung (100), umfassend:
ein Trägergestell (1);
einen Schleifring (2), drehbar an dem Trägergestell gelagert;
einen Antriebsmechanismus (3), an dem Trägergestell angebracht, wobei der Antriebsmechanismus ein Antriebsrad (31) umfasst;
ein Führungsrad (4), an dem Trägergestell angebracht; und
einen Endlosriemen (5), der eine geschlossene Peripherie umschließt, wobei der Endlosriemen mit dem Schleifring, dem Antriebsrad und dem Führungsrad in Kontakt ist, sodass das Antriebsrad den Schleifring und das Führungsrad über den Endlosriemen in Rotation versetzt,
wobei der Schleifring an einer Außenseite der Peripherie mit dem Endlosriemen in Kontakt ist.

2. Die CT-Scanvorrichtung nach Anspruch 1, wobei das Antriebsrad an der Außenseite der Peripherie mit dem Endlosriemen in Kontakt ist.

3. Die CT-Scanvorrichtung nach Anspruch 2, wobei das Führungsrad eine Mehrzahl von Führungsrädern umfasst, die um den Schleifring herum angeordnet sind, und wobei die Mehrzahl von Führungsrädern an einer Innenseite der Peripherie mit dem Endlosriemen in Kontakt sind.

4. Die CT-Scanvorrichtung nach Anspruch 3, wobei eines der Mehrzahl von Führungsrädern als ein Spannrad dient, das dazu eingerichtet ist, den Endlosriemen zu spannen.

5. Die CT-Scanvorrichtung nach Anspruch 3, ferner umfassend ein Spannrad (45), das dazu eingerichtet ist, den Endlosriemen zu spannen.

6. Die CT-Scanvorrichtung nach einem der Ansprüche 3 bis 5, wobei das Trägergestell umfasst:
einen Außenrahmen (11); und
einen Innenrahmen (12) mit zylindrischer Form, wobei der Innenrahmen durch eine Mehrzahl von Verbindungselementen im Außenrahmen befestigt ist, wobei der Schleifring über einen Lagermechanismus drehbar im Innenrahmen gelagert ist und sich axial aus dem Innenrahmen heraus erstreckt, um einen vorspringenden Abschnitt (21) zu bilden, und wobei der vorspringende Abschnitt mit dem Endlosriemen verbunden ist.

7. Die CT-Scanvorrichtung nach Anspruch 6, wobei der Antriebsmechanismus ferner umfasst:
einen Träger (32), der am Außenrahmen angebracht ist und dem Innenrahmen zugewandt ist; und
einen Motor (33), der auf einer Außenseite des Trägers gegenüber dem Innenrahmen angebracht ist, wobei eine Abtriebswelle (34) des Motors den Träger durchdringt und parallel zu einer Achse des Schleifrings verläuft, und wobei das Antriebsrad an der Abtriebswelle auf einer Innenseite des Trägers montiert ist.

8. Die CT-Scanvorrichtung nach Anspruch 7, wobei
die Mehrzahl von Führungsrädern ein erstes Führungsrad (41), ein zweites Führungsrad (42), ein drittes Führungsrad (43) und ein viertes Führungsrad (44) umfasst, die jeweils an den Eckpunkten eines Vierecks angeordnet sind,
der Endlosriemen zwischen dem ersten Führungsrad und dem zweiten Führungsrad mit dem Antriebsrad verbunden ist, und
das erste Führungsrad und das zweite Führungsrad drehbar an der Innenseite des Trägers gelagert sind.

9. Die CT-Scanvorrichtung nach Anspruch 8, wobei der Endlosriemen zwischen dem ersten Führungsrad und dem zweiten Führungsrad mit dem Schleifring verbunden ist, und wobei das erste Führungsrad drehbar an einem der Verbindungselemente gelagert ist.

10. Die CT-Scanvorrichtung nach Anspruch 9, wobei das vierte Führungsrad drehbar an einem anderen der Verbindungselemente gelagert ist.

11. Die CT-Scanvorrichtung nach einem der Ansprüche 6 bis 10, wobei der Endlosriemen aus einem Keilrippenriemen besteht und der vorspringende Abschnitt des Schleifrings mit einer Riemenscheibe (22) versehen ist, die mit dem Keilrippenriemen zusammenwirkt.

12. Die CT-Scanvorrichtung nach einem der Ansprüche 1 bis 11, wobei eine Röntgenröhre, die dazu eingerichtet ist, einen Röntgenstrahl auszusenden, und ein der Röntgenröhre radial gegenüberliegendes Detektorarray auf dem Schleifring montiert sind.

13. Die CT-Scanvorrichtung nach Anspruch 12, wobei der Endlosriemen an einem Abschnitt einer Außenumfangsfläche des Schleifrings in Kontakt ist, der zwischen der Röntgenröhre und dem Detektorarray liegt.

## Revendications

1. Appareil de tomographie assistée par ordinateur (100), comprenant:
un premier cadre de support (1);
une bague collectrice (2) montée à rotation sur le cadre de support;
un mécanisme d'entraînement (3) monté sur le cadre de support, dans lequel le mécanisme d'entraînement comprend une roue motrice (31);
une roue de guidage (4) montée à rotation sur le cadre de support; et
une courroie sans fin (5) renfermant une périphérie fermée, dans lequel la courroie sans fil est en contact avec la bague collectrice, la roue motrice et la roue de guidage, de manière à ce que la roue motrice entraîne la bague collectrice et la roue de guidage pour qu'elles soient mise en rotation par la courroie sans fin,
dans lequel la bague collectrice st en contact avec la courroie sans fin sur un côté extérieur de la périphérie.

2. Appareil de tomographie assistée par ordinateur selon le revendication 2, dans lequel la roue motrice est en contact avec la courroie sans fin sur un côté extérieur de la périphérie.

3. Appareil de tomographie assistée par ordinateur selon la revendication 2, dans lequel la roue de guidage comprend une pluralité de roues de guidage disposées autour de la bague collectrice, et la pluralité de roues de guidage est en contact avec la courroie sans fin sur un côté intérieur de la périphérie.

4. Appareil de tomographie assistée par ordinateur selon la revendication 3, dans lequel l'une parmi la pluralité des roues de guidage sert de roue de mise sous tension conçue pour tendre la courroie sans fin.

5. Appareil de tomographie assistée par ordinateur selon la revendication 3, comprenant en outre une roue de mise (45) conçue pour tendre la courroie sans fin.

6. Appareil de tomographie assistée par ordinateur selon l'une quelconque des revendications 3 à 5, dans lequel le cadre de support comprend:
un support extérieur (11); et
un cadre intérieur (12) se présentant forme de cylindre, dans lequel le cadre intérieur est fixé dans le cadre extérieur par l'intermédiaire d'une pluralité d'éléments de liaison, la bague collectrice est montée à rotation dans le cadre intérieur par l'intermédiaire d'un mécanisme de roulement et fait saillie axialement du cadre intérieur pour former une partie saillante (21), et la partie saillante est combinée avec la courroie sans fin.

7. Appareil de tomographie assistée par ordinateur selon la revendication 6, dans lequel le mécanisme d'entraînement comprend en outre:
une surface d'appui (32) montée sur le cadre extérieur et faisant face au cadre intérieur; et
un moteur (33) monté sur un côté extérieur de la surface d'appui en regard du cadre intérieur, dans lequel un arbre de sortie (34) du moteur traverse la surface d'appui et s'étend parallèlement à un axe de la bague collectrice, et la roue motrice est montée sur l'arbre de sortie sur un côté intérieur de la surface d'appui.

8. Appareil de tomographie assistée par ordinateur selon la revendication 7, dans lequel
la pluralité de roues de guidage comprend une première roue de guidage (41), une deuxième roue de guidage (42), une troisième roue de guidage (43) et une quatrième roue de guidage (44) respectivement situées aux sommets d'un quadrangle,
la courroie sans fin entre la première roue de guidage et la deuxième roue de guidage est combinée à la roue motrice, et
la première roue de guidage et la deuxième roue de guidage sont montées à rotation sur la face intérieure de la surface d'appui.

9. Appareil de tomographie assistée par ordinateur selon la revendication 8, dans lequel la courroie sans fin entre la première roue de guidage et la deuxième roue de guidage est combinée avec la bague collectrice, et
la première roue de guidage est montée à rotation sur l'un des éléments de liaison.

10. Appareil de tomographie assistée par ordinateur selon la revendication 9, dans lequel la quatrième roue de guidage est montée à rotation sur un autre élément parmi les éléments de liaison.

11. Appareil de tomographie assistée par ordinateur selon l'une quelconque des revendications 6 à 10, dans lequel la courroie sans fin est constituée d'une courroie à nervures multiples, et la partie saillante de la bague collectrice est pourvue d'un réa (22) coopérant avec la courroie à nervures multiples.

12. Appareil de tomographie assistée par ordinateur selon l'une quelconque des revendications 1 à 11, dans lequel un tube à rayons X conçu pour émettre des rayons X et un détecteur radialement opposé au tube à rayons X sont montés sur la bague collectrice.

13. Appareil de tomographie assistée par ordinateur selon la revendication 12, dans lequel
la courroie sans fin est en contact avec une partie d'une circonférence extérieure de la bague collectrice située entre le tube de rayons X et l'ensemble de détection.
